# EUROPEAN PATENT APPLICATION

(11) **EP 4 197 564 A1**
(43) Date of publication of application: **21.06.2023**
(21) Application number: 22208499.8
(22) Date of filing: 21.11.2022
(51) Int. Cl.: A61L 2/20, A61G 10/00, A61L 2/24

(54) **OZONE STERILIZATION DEVICE**

(30) Priority: 15.12.2021 JP 2021202957
(71) Applicant: KONICA MINOLTA, INC., Tokyo 100-7015 (JP); Tamura Teco Co., Ltd., Higashiosaka-shi, Osaka 577-0012 (JP)
(72) Inventor: FUJIMORI, Harumitsu, Tokyo, 100-7015 (JP); SAKATA, Satoshi, Tokyo, 100-7015 (JP); TAKAHASHI, Atsushi, Tokyo, 100-7015 (JP); FUKUDA, Yoshiyuki, Osaka, 577-0012 (JP)
(74) Representative: Gille Hrabal Partnerschaftsgesellschaft mbB Patentanwälte

(57) **Abstract**

An ozone sterilization device includes: an ozone generator that generates ozone gas; an ozone concentration measurement unit that measures an ozone concentration in air; a communication unit that performs data communication with an external recording device; an acquisition unit that acquires acquisition data at a predetermined acquisition timing, the acquisition data including the ozone concentration measured by the ozone concentration measurement unit; and a storage controller that stores the acquisition data in the external recording device via the communication unit.

## Description

### TECHNICAL FIELD

The present invention relates to an ozone sterilization device.

### DESCRIPTION OF THE RELATED ART

Conventionally, there have been proposed technologies using ozone to sterilize protective clothing worn by paramedics, white coats worn by doctors and the like at hospitals to which an injured or sick is transported, and treatment instruments that can be used repeatedly, etc. for the purpose of preventing secondary infection during emergency transport and treatment of the injured or sick who has been infected with a virus or other infectious agent.

Regarding such technologies, Patent No. JP 4762331 B2 discloses a disinfection device for returned paramedic that supplies ozone gas to a sealable disinfection room where the returned paramedic enters, calculates the CT value, that is a product of the time period spent by the paramedic in the disinfection room and the ozone concentration in the disinfection room, and notifies the paramedic of the fact that the CT value has reached a predetermined value.

Patent No. JP 5124536 B2 discloses an ozone sterilization management device that resets a measured CT value upon detection of start of a sterilization process, adds a product of the ozone concentration and a sampling interval (or an actual time interval) to the measured CT value, and, when the measured CT value is equal to or more than a judgment CT value for determining completion of the sterilization process, finishes the sterilization process.

Patent No. JP 5538037 B2 discloses an ozone sterilization management device that indicates a calculated CT value in multiple steps between 0% and 100% with respect to a set CT value by illumination of an indicator light, and, when a reset button and a down button for decreasing the CT set value are pressed at the same time, sets the set CT value to 0.

### SUMMARY OF THE INVENTION

However, when ozone gas is generated at a low concentration (for example, 0.1 ppm or less), the ozone sterilization device needs to be run continuously for a long time to achieve the target CT value. In such a case, the ozone concentration in the room where the ozone sterilization device is placed temporarily decreases when a human enters or exits the room. When using the ozone sterilization device described in Patent No. JP 4762331 B2, JP 5124536 B2, or JP 5538037 B2 that notifies a user of the CT value only, the user cannot correctly grasp the situation where the ozone concentration has temporarily decreased.

The present invention was made in view of the above problems, for the purpose of providing an ozone sterilization device that can manage sterilization with ozone more effectively.

To achieve at least one of the above-mentioned objects, according to an aspect of the present invention, an ozone sterilization device includes:
an ozone generator that generates ozone gas;
an ozone concentration measurement unit that measures an ozone concentration in air;
a communication unit that performs data communication with an external recording device;
an acquisition unit that acquires acquisition data at a predetermined acquisition timing, the acquisition data including the ozone concentration measured by the ozone concentration measurement unit; and
a storage controller that stores the acquisition data in the external recording device via the communication unit.

To achieve at least one of the above-mentioned objects, according to another aspect of the present invention, an ozone sterilization device includes:
an ozone generator that generates ozone gas;
an ozone concentration measurement unit that measures an ozone concentration in air;
a time measurement unit that measures an elapsed time from start of generating ozone gas by the ozone generator;
a calculator that calculates a CT value based on the ozone concentration measured by the ozone concentration measurement unit and the elapsed time measured by the time measurement unit;
a communication unit that performs data communication with an external recording device;
an acquisition unit that acquires acquisition data at a predetermined acquisition timing, the acquisition data including at least one of the ozone concentration measured by the ozone concentration measurement unit and the CT value calculated by the calculator; and
a storage controller that stores the acquisition data in the external recording device via the communication unit.

Preferably, the ozone sterilization device may further include:
a human detecting sensor that detects entry and exit of a human; and
a detector that detects an abnormality in the ozone sterilization device,
wherein the storage controller stores, as the acquisition data, data in which the at least one of a detection result by the human detecting sensor and a detection result by the detector are associated with the at least one of the ozone concentration measured by the ozone concentration measurement unit and the CT value calculated by the calculator in the external recording device.

Preferably, in the ozone sterilization device,
the acquisition unit acquires a detection result by the human detecting sensor and a detection result by the detector at a predetermined time interval, and,
upon the acquisition unit acquiring a detection result that the human detecting sensor has not detected human entry or exit and a detection result that the detector has not detected an abnormality continually for a predetermined number of times, the storage controller stores the acquisition data in the external recording device.

Preferably, the ozone sterilization device may further include:
a display; and
a controller that causes the display to display the CT value calculated by the calculator.

Preferably, in the ozone sterilization device, the storage controller stores the acquisition data in the external recording device continually at a predetermined time interval.

Preferably, in the ozone sterilization device, the predetermined time interval is a time period in minute.

Preferably, in the ozone sterilization device, the predetermined time interval is set to any value.

Preferably, in the ozone sterilization device, the communication unit performs data communication by a wireless communication method.

Preferably, in the ozone sterilization device, the storage controller stores the acquisition data in a cloud server as the external recording device.

Preferably, in the ozone sterilization device, the communication unit performs data communication by a wired communication method.

Preferably, in the ozone sterilization device, the storage controller stores the acquisition data in a portable storage medium as the external recording device.

Preferably, the ozone sterilization device may further include:
a lid that is configured to cover the portable storage medium; and
a main housing to which the lid is attached,
and the lid is detachable from the main housing using a dedicated tool.

Preferably, in the ozone sterilization device, the ozone generator generates ozone gas at a concentration applicable to a manned environment.

Preferably, in the ozone sterilization device, the storage controller outputs a communication clock signal only when the communication unit performs data communication with the external recording device.

Preferably, in the ozone sterilization device, the storage controller collectively stores the acquisition data acquired multiple times during a predetermined period in the external recording device.

### BRIEF DESCRIPTION OF THE DRAWINGS

The advantages and features provided by one or more embodiments of the invention will become more fully understood from the detailed description given hereinbelow and the appended drawings which are given by way of illustration only, and thus are no intended as a definition of the limits of the present invention, wherein:
FIG. 1 is a schematic diagram of an ozone sterilization device;
FIG. 2 is a block diagram showing a functional configuration of the ozone sterilization device;
FIG. 3 is a diagram showing an example of an operation unit and a display;
FIG. 4 is a diagram showing an example of a port of a communication unit;
FIG. 5A is a structural diagram of a lid and a main housing near the lid;
FIG. 5B is a cross-sectional view of FIG. 5A cut at VB-VB;
FIG. 6 is a flowchart showing steps in the log storing process;
FIG. 7A is a diagram showing an example of stored date and time of storage timings and acquisition data;
FIG. 7B is a diagram showing an example of stored date and time of storage timings and acquisition data;
FIG. 8 is a diagram explaining an example of a storing process of acquisition data in an external recording device using the wireless communication method of Modification Example 1;
FIG. 9 is a flowchart showing steps in the second data storing process according to Modification Example 2;
FIG. 10 is a diagram showing an example of stored date and time of storage timings and acquisition data according to Modification Example 2;
FIG. 11 is a flowchart showing steps in the third data storing process according to Modification Example 3; and
FIG. 12 is a flowchart showing steps in the fourth second data storing process according to Modification Example 4.

### DETAILED DESCRIPTION

Hereinafter, preferred embodiments of the present invention will be explained with reference to the drawings. However, the scope of the invention is not limited to the disclosed embodiments.

### (Configuration of Ozone Sterilization Device 100)

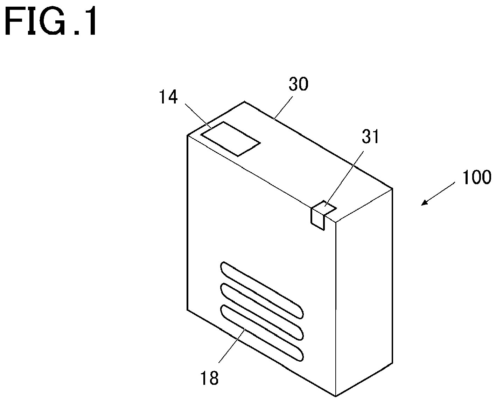

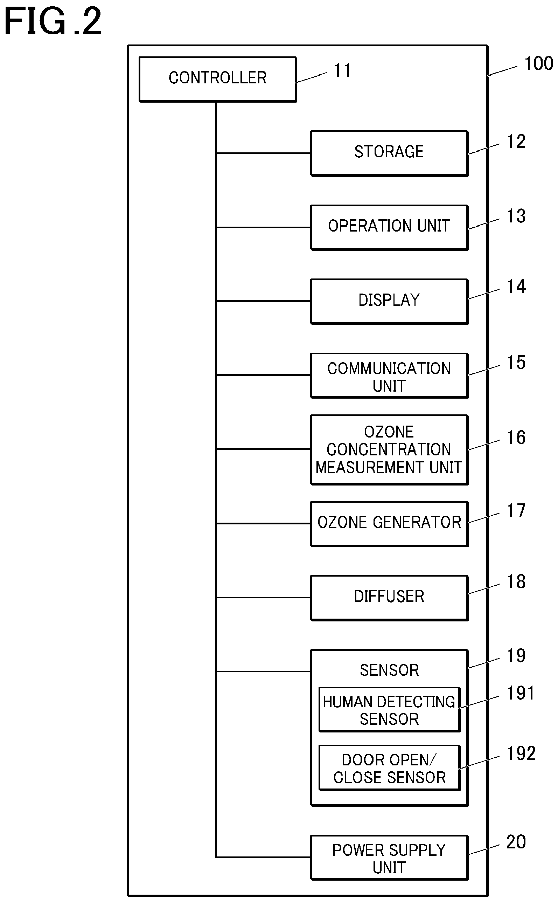

As illustrated in FIG. 1 and FIG. 2, an ozone sterilization device 100 according to a preferred embodiment of the present invention includes a controller 11, a storage 12, an operation unit 13, a display 14, a communication unit 15, an ozone concentration measurement unit 16, an ozone generator 17, a diffuser 18, a sensor 19, a power supply unit 20, a main housing 30, a lid 31 that is attached to the main housing 30, and the like.

The controller 11 controls behavior of the ozone sterilization device 100. Specifically, the controller 11 includes a CPU (Central Processing Unit), an ROM (Read Only Memory), an RAM (Random Access Memory), and the like, and comprehensively controls each part of the ozone sterilization device 100 through cooperation of the CPU and a computer program that is stored in the ROM or the storage 12 and expanded in a working area of the RAM.

The controller 11 also measures an elapsed time from the start of ozone gas generation by the ozone generator 17. Here, the controller 11 functions as a time measurement unit.

The controller 11 also calculates the CT value based on the ozone concentration measured by the ozone concentration measurement unit 16 and the elapsed time measured by the time measurement unit. Here, the controller 11 functions as a calculator.

The controller 11 also acquires, at predetermined acquisition timings, acquisition data including the ozone concentration measured by the ozone concentration measurement unit 16 and/or the CT value calculated by the calculator. Here, the controller 11 functions as an acquisition unit. Here, an interval between the predetermined acquisition timings only needs to be shorter than the interval between storage timings described later.

The controller 11 also causes the acquisition data to be stored in an external recording device (described later) via the communication unit 15. Here, the controller 11 functions as a storage controller.

The storage 12 is configured with, for example, a hard disk drive (HDD), a semiconductor memory, and the like, where data and programs can be written and read.

The operation unit 13 receives an input operation by a user and outputs an operation signal to the controller 11 in response to the input operation.

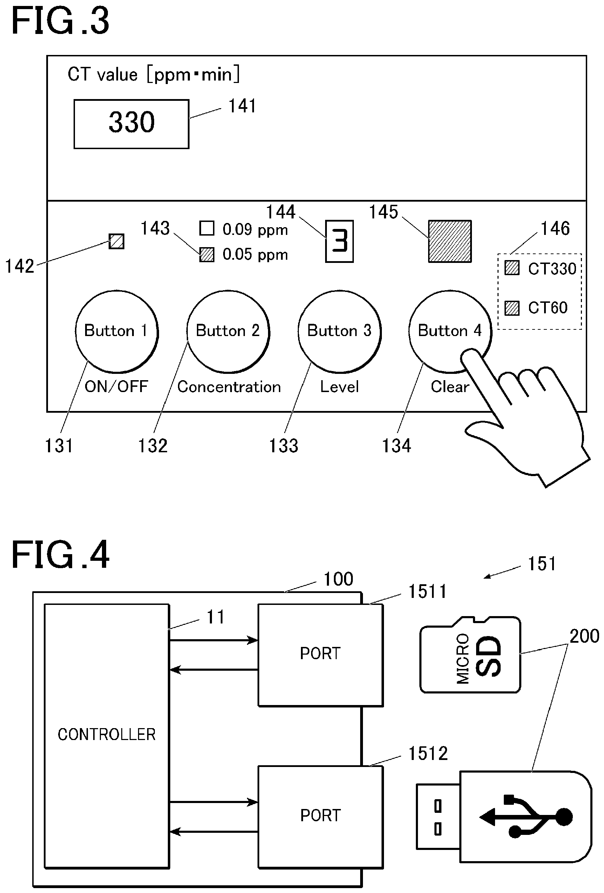

FIG. 3 shows an example of the operation unit 13 and the display 14.

As illustrated in FIG. 3, the operation unit 13 includes an ON/OFF button 131, a Concentration button 132, a Level button 133, and a Clear button 134.

The ON/OFF button 131 receives an input operation as to start or stop of an ozone generating process by the ozone sterilization device 100.

The Concentration button 132 receives an input operation as to setting of the target ozone concentration in an environment where the ozone sterilization device 100 is placed. In the example illustrated in FIG. 3, the target ozone concentration can be selected to be either 0.09 ppm or 0.05 ppm. The target ozone concentration may be configured to be set to any value as long as it is low enough to be applied to a manned environment (specifically, 0.10 ppm or less, which is an acceptable concentration for the human body, or 0.05 ppm or less).

The Level button 133 receives an input operation as to setting of the amount of ozone to be generated by the ozone generator 17. In the example illustrated in FIG. 3, the Level button 133 receives an input operation of 1 to 5, corresponding to five levels as to setting of the amount of ozone to be generated.

The Clear button 134 receives an input operation as to clearance of maintenance timing notification (described later) of the ozone sterilization device 100.

The display 14 displays various kinds of information depending on instructions based on display control signals that are input by the controller 11.

As illustrated in FIG. 3, the display 14 has a calculated CT value display 141, an ON/OFF display 142, a Concentration display 143, a Level display 144, a Clear display 145, and a reached CT value display 146.

As illustrated in FIG. 3, the ON/OFF button 131 and the ON/OFF display142, the Concentration button 132 and the Concentration display 143, the Level button 133 and the Level display 144, and the Clear button 134 and the Clear display 145 are each arranged correspondingly in a one-to-one pair in the vertical direction in the drawing.

The calculated CT value display 141 displays the CT value calculated by the controller 11 (calculator).

When the ON/OFF button 131 has been pressed so that the ozone sterilization device 100 is turned on (when the ozone generating process is started), an LED in the ON/OFF display 142 emits green light, for example.

In the Concentration display 143, an LED corresponding to the target ozone concentration selected upon pressing of the Concentration button 132 emits light. In the example illustrated in FIG. 3, the LED corresponding to 0.05 ppm emits light.

The Level display 144 displays the level of the amount of ozone to be generated selected upon pressing of the Level button 133. In the example illustrated in FIG. 3, "3" is displayed out of the ozone generation levels of 1 to 5.

As the maintenance timing notification indicating that it is time to perform maintenance of the ozone sterilization device 100, the LED in the Clear display 145 emits red light. When the user performs maintenance and presses the Clear button 134, the LED in the Clear display 145 emits blue light.

When the CT value calculated by the controller 11 (the calculator) reaches a predetermined CT value, an LED in the reached CT value display 146 emits light corresponding to the predetermined CT value. In the example illustrated in FIG. 3, the LEDs corresponding to a CT value of 60 and a CT value of 330 both emit light.

The communication unit 15 performs data communication with an external device(s) using a predetermined communication protocol under the control by the controller 11. Specifically, the communication unit 15 is a communication interface having a communication IC (integrated circuit) and a communication connector. The communication unit 15 performs data communication with the external device that is connected by wire or wirelessly through communication networks such as a LAN (local area network), a WAN (wide area network), the Internet, and the like.

As illustrated in FIG. 4, the communication unit 15 has a port 151 where a portable storage medium 200, which is the external recording device, can be inserted. In the example illustrated in FIG. 4, the communication unit 15 has a port 1511 where a micro SD card (registered trademark) as the portable storage medium 200 can be inserted and a port 1512 where a USB (Universal Serial Bus) (registered trademark) memory as the portable storage medium 200 can be inserted.

The communication unit 15 may have either one of the port 1511 and the port 1512.

A portable storage medium other than the micro SD card or the USB memory may be configured to be inserted into the communication unit 15.

The portable storage medium 200 stores the date and time of the storage timing (described later) and the acquisition data (described later) including the ozone concentration measured at the storage timing and the calculated CT value.

The portable storage medium 200 can be removed from the port 151 after the ozone sterilization device 100 has been turned off.

The lid 31 covers the port 151 and the portable storage medium 200 inserted in the port 151.

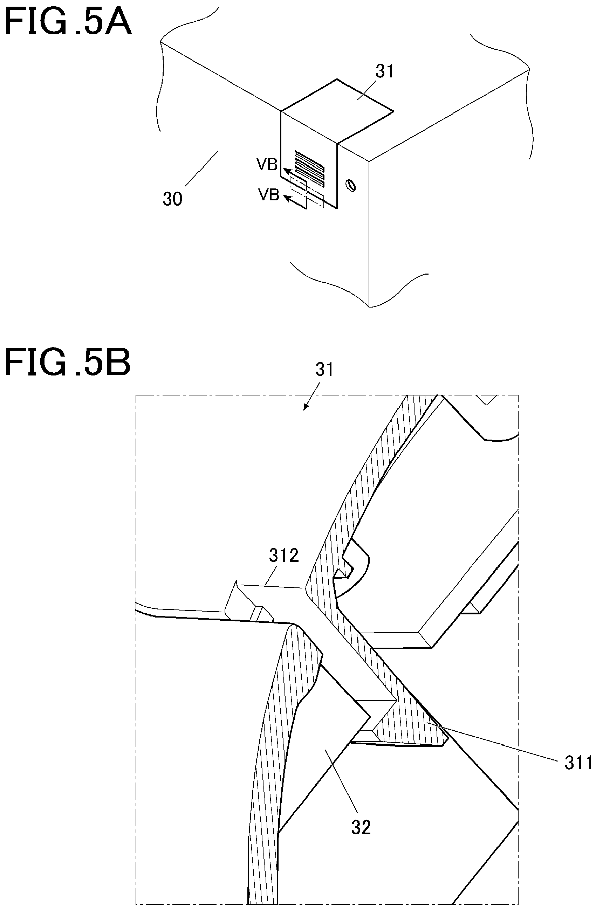

FIG. 5A shows a structural view of the lid 31 and the main housing 30 near the lid 31. FIG. 5B shows a cross-sectional view of FIG. 5A at VB-VB.

As illustrated in FIG. 5B, the lid 31 has a projection 311 and an opening 312. The main housing 30 has an engaging portion 32 that engages with the projection 311.

The projection 311 engages with the engaging portion 32 using a snap fit method.

The opening 312 is used to disengage the projection 311 from the engaging portion 32, and the size of the opening is set such that a person's finger cannot enter the opening.

The lid 31 can be removed from the main housing 30 using a small dedicated tool (for example, a flat-blade screwdriver) that can be inserted into the opening 312. The dedicated tool is inserted in the opening 312 so as to push the projection 311 upward, thereby disengages the projection 311 and the engaging portion 32 and removes the lid 31 from the main housing 30.

By using the lid 31 and the main housing 30 having the above structure, the portable storage medium 200 in which data are stored cannot be easily removed even when the ozone sterilization device 100 is placed in an environment where an unspecified large number of people are present.

The ozone concentration measurement unit 16 measures the ozone concentration in the air in the environment where the ozone sterilization device 100 is placed using an ultraviolet absorption method or a semiconductor thin-film method, and outputs the measurement result to the controller 11. The measurement method of the ozone concentration is not limited to the above.

The ozone generator 17 generates ozone gas of a low concentration that is applicable to a manned environment (specifically, 0.10 ppm or less, which is an acceptable concentration for the human body or 0.05 ppm or less) using a silent discharge method, an ultraviolet light lamp method, or a creepage discharge method under the control of the controller 11. The method of generating ozone gas is not limited to the above.

The diffuser 18 diffuses the ozone gas generated by the ozone generator 17 in the environment where the ozone sterilization device 100 is placed.

The diffuser 18 is, for example, a fan motor having vanes that rotate to generate an air flow and to diffuse the generated ozone gas.

The diffuser 18 may diffuse the generated ozone gas using an upward air flow resulting from air warmed in the ozone sterilization device 100.

The ozone sterilization device 100 does not need to have the diffuser 18 when there is an air flow in the environment where the ozone sterilization device 100 is placed.

The sensor 19 includes a human detecting sensor 191 and a door open/close sensor 192.

The human detecting sensor 192 senses a human, detects entry and exit of the human in the environment where the ozone sterilization device 100 is placed, and outputs the detection result(s) to the controller 11. The detection results are temporarily stored in the RAM of the controller 11.

The door open/close sensor 192 is provided on the exterior of an intake door and/or an exhaust door of the diffuser 18, detects the open/close status of the door(s), and outputs the detection results to the controller 11. The detection results are temporarily stored in the RAM of the controller 11.

The power supply unit 20 converts the power supplied from the outside into the power required by each part of the ozone sterilization device 100 and supplies the converted power to each part.

### (Behavior of Ozone Sterilization Device 100)

Next, behavior of the ozone sterilization device 100 will be described.

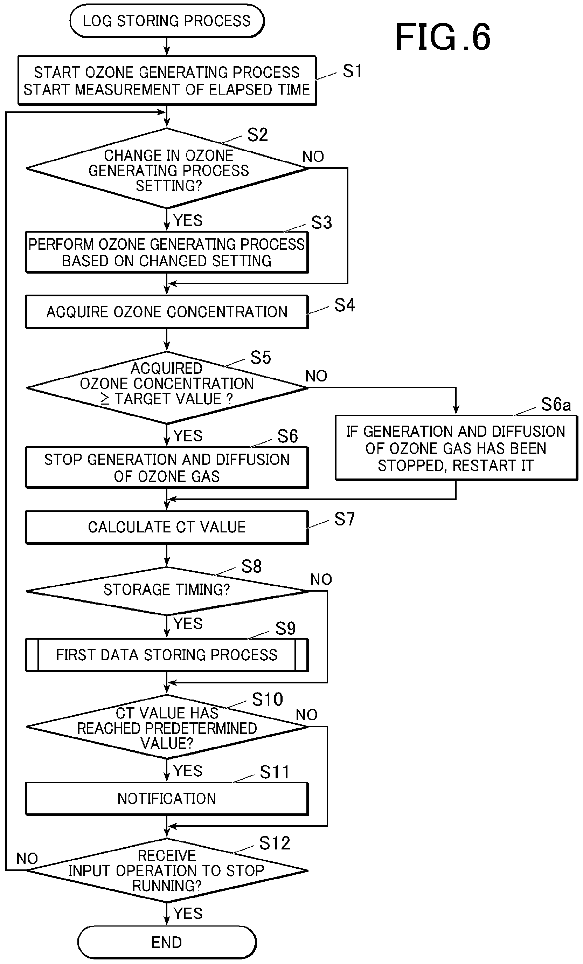

When receiving the input operation as to start of the ozone generating process by the ozone sterilization device 100 upon pressing of the ON/OFF button 131 by the user, the controller 11 starts a log storing process illustrated in FIG. 6.

First, the controller 11 starts the ozone generating process and starts measurement of the elapsed time from the start of the ozone generating process (step S1).

The ozone generating process includes controlling the ozone generator 17 to generate ozone gas, controlling the diffuser 18 to diffuse the generated ozone gas, and displaying current settings in the ozone generating process on the display 14.

In displaying the current settings, the controller 11 causes the LED in the ON/OFF display 142 to emit light depending on the input operation as to start or stop of the ozone generation that is set in response to pressing of the ON/OFF button 131. The controller 11 also causes the LED in the Concentration display 143 to emit light corresponding to the target ozone concentration in the environment where the ozone sterilization device 100 is placed that is selected upon pressing the Concentration button 132. The controller 11 also causes the Level display 144 to display the level of the amount of ozone to be generated by the ozone generator 17 that is set upon pressing of the Level button 133. The controller 11 also causes the LED in the Clear display 145 to emit red light when it is time to perform maintenance of the ozone sterilization device 100, and causes the LED in the Clear display 145 to emit blue light otherwise.

Next, the controller 11 determines whether or not the settings in the ozone generating process have been changed by the user's input operation via the operation unit 13 (step S2). The settings in the ozone generating process include a setting of the target ozone concentration in the environment where the ozone sterilization device 100 is placed, a setting of the amount level of ozone to be generated by the ozone generator 17, and clearance of the maintenance timing notification.

If at least one of the settings in the ozone generating process has been changed (step S2; YES), the controller 11 performs the ozone generating process based on the settings changed in step S2 (step S3).

After the process of step S3 or if the settings in the ozone generating process have not been changed (step S2; NO), the controller 11 controls the ozone concentration measurement unit 16 to acquire the ozone concentration in the air in the environment where the ozone sterilization device 100 is placed (step S4).

Next, the controller 11 determines whether or not the ozone concentration acquired in step S4 is equal to or more than the set target ozone concentration (step S5).

The controller 11 may acquire the ozone concentration every predetermined time period (for example, every several seconds) in step S4, calculate the average of the acquired results, and determine whether or not the calculated average is equal to or more than the set target ozone concentration in step S5.

If the acquired ozone concentration is equal to or more than the set target ozone concentration (step S5; YES), the controller 11 stops the ozone gas generation by the ozone generator 17 and the ozone gas diffusion by the diffuser 18 (step S6).

After the process of step S6 or if the acquired ozone concentration is less than the set target ozone concentration (step S5; NO), the controller 11 calculates the CT value, which is a product of the ozone concentration acquired in step S4 and the elapsed time from the start of the ozone generating process (step S7). If the process of step S5 is performed for the first time in the log storing process, the process of step S6a (described later) is not performed.

Next, the controller 11 determines whether or not it is the storage timing to store the ozone concentration acquired in step S4 and the CT value calculated in step S7 (step S8). The storage timing is the time when a predetermined time period (for example, a time period in minute, and specifically one minute) has passed since the ozone concentration and the previous CT value were previously stored.

Because the CT value [ppm × min] is the product of the ozone concentration [ppm] and the ozone exposure time (fumigation time) [min], the ozone concentrations and CT values stored at minute intervals allow the user to use the stored ozone concentrations and CT values to further improve the work efficiency in verifying the CT values.

The above predetermined time period is not limited to one minute, but may be, for example, ten seconds, five minutes, or the like.

The above predetermined time period may also be configured to be set to any value by the user via the communication unit 15.

If it is the storage timing (step S8; YES), the controller 11 performs a first data storing process (step S9). Specifically, the controller 11 stores, in the portable storage medium 200 inserted in the port 151, the date and time of the storage timing, the ozone concentration acquired in step S4, and the CT value calculated in step S7.

Here, the ozone concentration acquired in step S4 and the CT value calculated in step S7 are the acquisition data.

In step S9, the controller 11 may store the ozone concentration acquired at the storage timing (for example, at the time when one minute has passed since the previous ozone concentration and the previous CT value were stored) or may store an average value of measurement results of the ozone concentration acquired every predetermined time period (for example, several seconds) between storage timings.

Alternatively, in step S9, the controller 11 may store one of the ozone concentration acquired in step S4 and the CT value calculated in step S7 as the acquisition data.

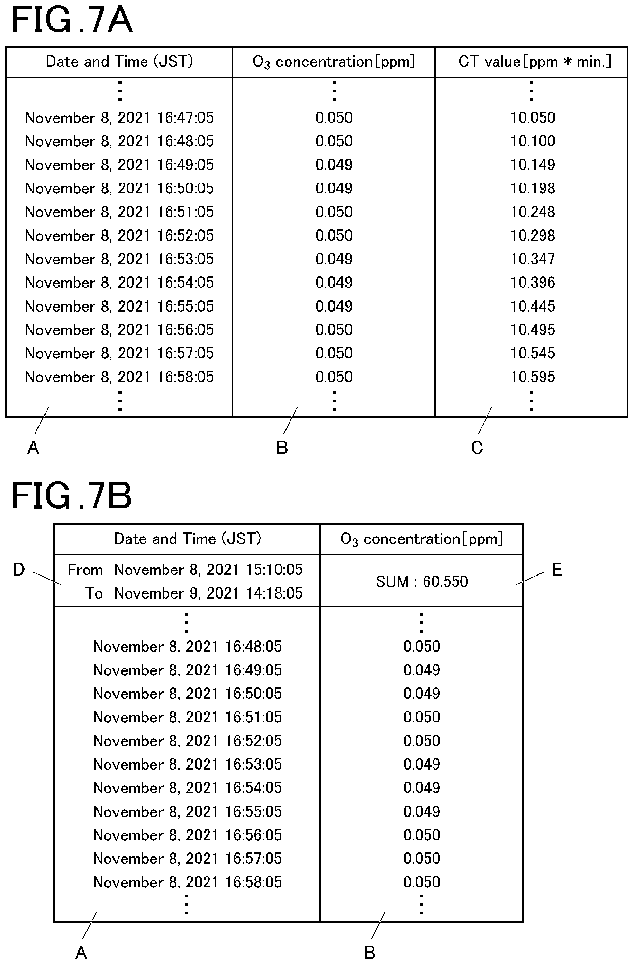

FIG. 7A and FIG. 7B show examples of date and time of storage timing and acquisition data stored in the portable storage medium 200.

In the example illustrated in FIG. 7A, the ozone (O₃) concentrations (B) acquired at respective storage timings (A) and the respective calculated CT values (C) are stored.

In the example illustrated in FIG. 7B, the ozone concentrations (B) acquired at the respective storage timings (A), the date and time of the start and the end of the ozone generating process (D), and the total value of the ozone concentrations acquired during the period from the start to the end (E) are stored.

In the examples illustrated in FIG. 7A and FIG. 7B, the storage timing is one minute after the previous storage of the ozone concentration and the CT value. Therefore, the stored acquisition data are time series data in which data points acquired continually at one-minute intervals are arranged sequentially.

In other words, the controller 11 stores the acquisition data in the external recording device continually at a predetermined time interval. The controller 11 may control the external recording device to store the acquisition data, or may send the acquisition data to the external recording device such that the acquisition data is stored there.

Because the acquisition data acquired at the predetermined acquisition timings as described above are stored in the external recording device, the user can be provided with the acquisition data via the external recording device.

Also, because the acquisition data stored in the external recording device are time series data including data points acquired continually at predetermined time intervals, the user can be provided with the acquisition data such that calculation and verification of the CT values are possible.

As described above, the controller 11 stores the acquisition data in the portable storage medium 200 inserted in the port 151 connected to the controller 11 by a wired communication method. Therefore, even when a device (for example, a medical device defined in Article 2 of Japanese Pharmaceutical Medical Device Act) that is poorly tolerant to near-field electromagnetic fields of a radio frequency (RF) wireless communication device is placed in the room where the ozone sterilization device 100 is placed, the acquisition data can be stored without affecting the device.

Because the acquisition data are stored in the portable storage medium 200, the user can remove only the portable storage medium 200 where the acquisition data are stored from the ozone sterilization device 100 and verify the CT values in a place outside of the room where the ozone sterilization device 100 is placed.

After the process of step S9 or if it is not the storage timing (step S8; NO), the controller 11 determines whether or not the CT value calculated in step S7 has reached the predetermined CT value (step S 10). In the example illustrated in FIG. 3, the predetermined CT value is either CT value 60 or CT value 330.

If the calculated CT value has reached the predetermined CT value (step S10; YES), the controller 11 causes the LED corresponding to the predetermined CT value in the reached CT value display 146 to emit light and notifies the user that the calculated CT value has reached the predetermined CT value.

After the process of step S11, or if the calculated CT value has not reached the predetermined CT value (step S10; NO), the controller 11 determines whether or not the ON/OFF button 131 has been pressed by the user and has received the input operation as to stop of the ozone generating process by the ozone sterilization device 100 (step S12).

If the input operation as to stop of the ozone generating process by the ozone sterilization device 100 has been received (step S12; YES), the controller 11 finishes the log storing process.

If the input operation as to stop of the ozone generating process by the ozone sterilization device 100 has not been received (step S12; NO), the controller 11 moves the process to step S2.

If the processes of steps S2 to S5 have been performed two or more times, the ozone concentration acquired in step S4 is less than the set target ozone concentration (step S5; NO), and the diffuser 18 has stopped diffusing ozone gas, the controller 11 restarts ozone gas generation by the ozone generator 17 and ozone gas diffusion by the diffuser 18 (step S6a).

### (Modification Example 1)

Next, Modification Example 1 of the above embodiment will be described.

In the following, differences from the above embodiment will be mainly described. The configuration of the ozone sterilization device 100 in Modification Example 1 is the same as the ozone sterilization device 100 of the above embodiment.

In the first data storing process (step S9) of the log storing process of Modification Example 1, the controller 11 stores the date and time of the storage timing and acquisition data in the external recording device by a wireless communication method via the communication unit 15.

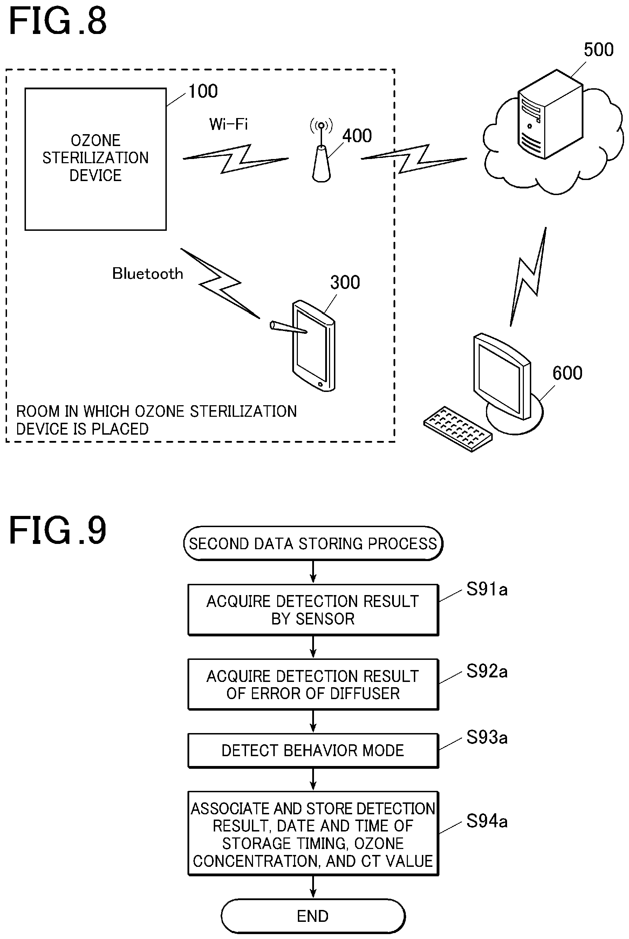

For example, as illustrated in FIG. 8, when the ozone sterilization device 100 and a dedicated wireless terminal 300 (the external recording device) in the room where the ozone sterilization device 100 is placed are connected via the communication unit 15 using Bluetooth (registered trademark), the controller 11 stores the date and time of the storage timings and the acquisition data in the wireless terminal 300 in step S9 above.

As illustrated in FIG. 8, when the ozone sterilization device 100 and an IoT (Internet of Thing) gateway device 400 provided in the room where the ozone sterilization device 100 is placed are connected via the communication unit 15 using Wi-Fi (registered trademark), the controller 11 stores the date and time of the storage timing and the acquisition data in an external recording device (a data server 500) in the cloud via the IoT gateway device 400 in above step S9. The ozone sterilization device 100 is configured to allow a user to enter a password from a personal computer 600, to log in to the data server 500, and to view data stored on the data server 500 from anywhere.

When the acquisition data is stored in the external recording device by the wireless communication method via the communication unit 15 as described above, the user can easily acquire the stored acquisition data from the external recording device even when the ozone sterilization device 100 is placed at a high place that the user has difficulty in reaching.

When the acquisition data is stored in the data server 500 via the IoT gateway device 400, the user can check the stored acquisition data even when the user is not in the room where the ozone sterilization device 100 is placed.

### (Modification Example 2)

Next, Modification Example 2 of the above embodiment will be described.

In the following, differences from the above embodiment will be mainly described. The configuration of the ozone sterilization device 100 in Modification Example 2 is the same as the ozone sterilization device 100 of the above embodiment.

In Modification Example 2, the controller 11 performs a second data storing process illustrated in FIG. 9 instead of the first data storing process in the log storing process of the above embodiment.

In the second data storing process, the controller 11 first acquires the detection result by the human detecting sensor 191 and the detection result by the door open/close sensor 192 of the sensor 19 from the previous storage timing to the current storage timing (step S91a).

Here, the door open/close sensor 192 functions as a detector that detects an abnormality in the ozone sterilization device 100.

Next, the controller 11 acquires the detection result of whether or not an error has occurred in the behavior of the diffuser 18 during the period between the previous storage timing and the current storage timing (step S92a). The error in the behavior of the diffuser 18 is, for example, insufficient rotation of the fan motor.

Next, the controller 11 detects a current behavior mode of the ozone sterilization device 100 (step S93a).

The behavior mode of the ozone sterilization device 100 is one of the following: a warm-up mode immediately after turning on the power and the like, in which the ozone sterilization device 100 is preparing a normal behavior; a running mode in which the ozone generator 17 is controlled to generate ozone gas and the diffuser 18 is controlled to diffuse the generated ozone gas; a standby mode in which ozone gas is not generated and the diffuser 18 is in operation; and an error mode in which the ozone generator 17 and the diffuser 18 are stopped due to an abnormal ozone concentration measured by the ozone concentration measurement unit 16 and the like.

The behavior mode may also include the target ozone concentration in the environment where the ozone sterilization device 100 is placed that is set upon pressing of the Concentration button 132 and the ozone level to be generated by the ozone generator 17 that is set upon pressing of the Level button 133.

Here, the controller 11 acquires the detection result of whether or not an error has occurred in the behavior of the diffuser 18 and detects the behavior mode of the ozone sterilization device 100. Thus, the controller 11 functions as a detector that detects an abnormality in the ozone sterilization device 100.

Next, the controller 11 associates the detection result by the human detecting sensor 191 and the detection result by the door open/close sensor 192 acquired in step S91a, the detection result of an error in the behavior of the diffuser 18 acquired in step S92a, the detection result of the behavior mode of the ozone sterilization device 100 detected in step S93a, the date and time of the storage timing, the ozone concentration acquired in step S4 of the log storing process, and the CT value calculated in step S7, and stores them in the external recording device (step S94a). The controller 11 then finishes the second data storing process and moves the process to step S10 of the log storing process.

In this case, the acquisition data are the data associating the detection result by the human detecting sensor 191, the detection result by the door open/close sensor 192, the detection result of the error in the behavior of the diffuser 18, the detection result of the behavior mode of the ozone sterilization device 100, the measured ozone concentration, and the calculated CT value.

The external recording device may be a portable storage medium 200 inserted in the port 151, or may be an external recording device connected by the wireless communication method via the communication unit 15.

In step S94a of the above second data storing process, the controller 11 may associate either of the measured ozone concentration or the calculated CT value with the detection result by the human detecting sensor 191, the detection result by the door open/close sensor 192, the detection result of the error in the behavior of the diffuser 18, and the detection result of the behavior mode of the ozone sterilization device 100 and store them.

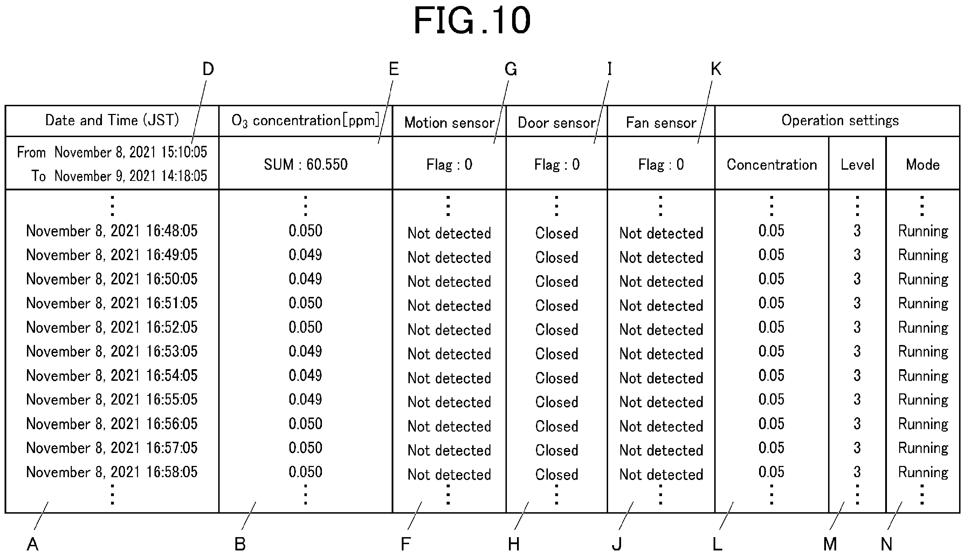

FIG. 10 shows an example of the date and time of the storage timing and acquisition data stored in step S94a of the above second data storing process.

In the example illustrated in FIG. 10, in the same manner as in the example illustrated in FIG. 7B, the ozone concentrations (B) acquired at the storage timings (A), the date and time of the start and the end of the ozone generating process (D), and the total value of the ozone concentrations measured during the period from the start to the end (E) are stored.

As illustrated in FIG. 10, the detection results by the human detecting sensor 191 (F) from the respective previous storage timings to the respective current storage timings are acquired at the respective storage timings (A) and stored. Specifically, as the detection result by the human detecting sensor 191 (F), "Detected" is stored if the human detecting sensor 191 has detected a human, and "Not detected" is stored if the human detecting sensor 191 has not detected a human.

Also, the number of times (G) the human detecting sensor 191 has detected a human from the date and time of the start to that of the end of the ozone generating process (D) is stored.

Furthermore, as illustrated in FIG. 10, the detection results by the door open/close sensor 192 (H) from the respective previous storage timings to the current storage timings are acquired at the respective storage timings (A) and stored. Specifically, as the detection result (H) by the door open/close sensor 192, "Open" is stored when the door open/close sensor 192 has detected opening of the intake door or the exhaust door of the diffuser 18, and "Closed" is stored when the door open/close sensor 192 has detected closing of the intake door or the exhaust door of the diffuser 18.

Also, the number of times (I) the door open/close sensor 192 has detected opening of the intake door or the exhaust door of the diffuser 18 from the date and time of the start to that of the end of the ozone generating process (D) is stored.

Furthermore, as illustrated in FIG. 10, the detection results (J) of an error (for example, insufficient rotation of the fan motor) in the behavior of the diffuser 18 from the previous storage timing to the current storage timing is acquired at each of the storage timings (A) and stored. Specifically, as the detection result (J) of the error in the behavior of the diffuser 18, "Detected" is stored when the error in the behavior of the diffuser 18 has been detected, and "Not detected" is stored when the error in the behavior of the diffuser 18 has not been detected.

Also, the number of times (K) the error in the behavior of the diffuser 18 has been detected from the date and time of the start to that of the end of the ozone generating process (D) is stored.

Furthermore, as illustrated in FIG. 10, the stored detection result of the behavior mode of the ozone sterilization device 100 detected at each of the storage timings (A) includes the target ozone concentration (L) in the environment where the ozone sterilization device 100 is placed, the ozone level (M) generated by the ozone generator 17, and the behavior mode (N). For example, when the behavior mode of the ozone sterilization device 100 is the running mode, "Running" is stored as the behavior mode (N).

Because the data (the acquisition data) associating the detection results by the sensor 19, the detection result of an error in the behavior of the diffuser 18, the detection result of the behavior mode of the ozone sterilization device 100, the acquired ozone concentration, and the calculated CT value are stored as described above, when there is a change such as a temporary decrease in the ozone concentration in the time series data of the ozone concentration, for example, it is possible to identify the cause of the decrease in the ozone concentration based on these detection results.

Specifically, when detection of a human by the human detecting sensor 191 is stored at the timing when the temporary decrease in the ozone concentration is stored, it can be assumed that the temporary decrease in the ozone concentration would have occurred because a human had entered or left the room where the ozone sterilization device 100 was placed.

### (Modification Example 3)

Next, Modification Example 3 of the above embodiment will be described.

In the following, differences from the above embodiment will be mainly described. The configuration of the ozone sterilization device 100 in Modification Example 3 is the same as the ozone sterilization device 100 of the above embodiment.

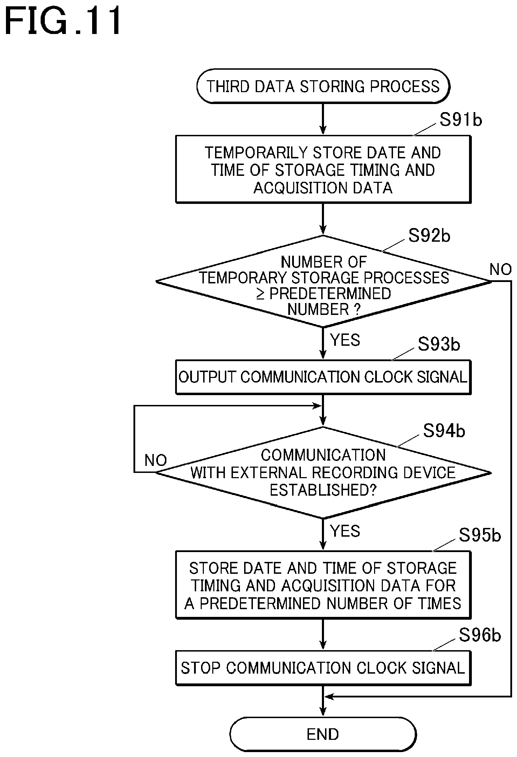

In Modification Example 3, the controller 11 performs a third data storing process illustrated in FIG. 11 instead of the first data storing process in the log storing process of the above embodiment.

In the third data storing process, first, the controller 11 stores the date and time of the storage timings and the acquisition data (the ozone concentration acquired in step S4 and the CT value calculated in step S7 in the log storing process) temporarily in the RAM of the controller 11 (step S91b).

Next, the controller 11 determines whether or not the date and time of the storage timings and the acquisition data have been temporarily stored for a predetermined number of times or more (for example, 15 times or more) in step S91b since the date and time of the storage timings and the acquisition data was previously stored in the external recording device (step S92b). The predetermined number can be set to any value.

If the date and time of the storage timings and the acquisition data have been temporarily stored for a predetermined number of times or more (step S92b; YES), the controller 11 outputs a communication clock signal in order to connect to the external recording device by wireless communication method via the communication unit 15 (step S93b).

Next, the controller 11 determines whether or not communication with the external recording device has been established (step S94b).

If communication with the external recording device has not been established (step S94b; NO), the controller 11 returns the process to step S94b. In other words, the controller 11 waits for establishment of the communication with the external recording device.

If communication with the external recording device has been established (step S94b; YES), the controller 11 collectively stores, in the external recording device, the date and time of the storage timings and acquisition data for a predetermined number of times that are temporarily stored in step S91b after the previous storage of the date and time of the storage timings and the acquisition data in the external recording device (step S95b).

Next, the controller 11 stops the communication clock signal (step S96b), finishes the third data storing process, and moves the process to step S10 of the log storing process.

As the date and time of the storage timings and the acquisition data temporarily stored for a predetermined number of times are collectively stored in the external recording device as described above, it is possible to reduce the frequency of communication between the ozone sterilization device 100 and the external recording device. In addition, because the communication clock signal is output only when data is stored in the external recording device, the power consumption of the controller 11 (CPU) can be suppressed. Since the ozone sterilization device 100 needs to run continuously for a long time to achieve the high CT value while generating a low concentration of ozone gas, it is desirable to reduce the power consumption of the controller 11 (CPU).

Alternatively, if the date and time of the storage timings and the acquisition data have been temporarily stored for less than a predetermined number of times (step S92b; NO), the controller 11 finishes the third data storing process, and moves to the process to step S10 of the log storing process.

### (Modification Example 4)

Next, Modification Example 4 of the above embodiment will be described.

In the following, differences from the above embodiment will be mainly described. The configuration of the ozone sterilization device 100 in Modification Example 4 is the same as the ozone sterilization device 100 of the above embodiment.

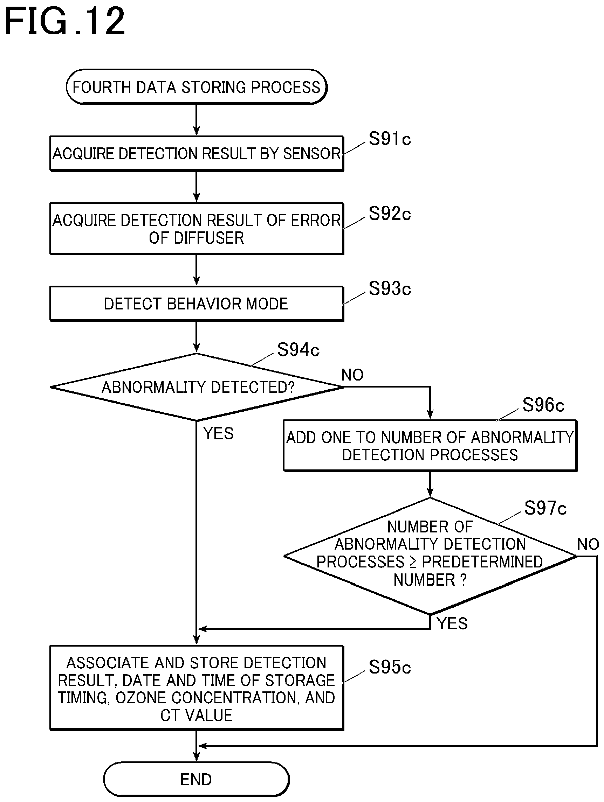

In Modification Example 4, the controller 11 performs a fourth data storing process illustrated in FIG. 12 instead of the first data storing process in the log storing process of the above embodiment.

In the fourth data storing process, first, the controller 11 performs processes of steps S91c to S93c that are the same as the processes of the steps S91a to S93a of the second data storing process.

Next, the controller 11 determines whether or not an abnormality has been detected based on the detection results in steps S91c to S93c (step S94c). Examples of the abnormality in these detection results include: detection of a human being by the human detecting sensor 191 in step S91c; detection of opening of the intake door or the exhaust door of the diffuser 18 by the door open/close sensor 192; detection of an error in the behavior of the diffuser 18 in step S92c; and detection in step S93c that the behavior mode of the ozone sterilization device 100 is the error mode.

Processes in steps S91c to S94c relate to an abnormality detection process.

If an abnormality has been detected in the detection results (step S94c; YES), the controller 11 associates the detection result by the human detecting sensor 191 and the detection result by the door open/close sensor 192 acquired in step S91c, the detection result of the error in the behavior of the diffuser 18 acquired in step S92c, the detection result of the behavior mode of the ozone sterilization device 100 detected in step S93c, the date and time of the storage timing, the ozone concentration acquired in step S4 of the log storing process, and the CT value calculated in step S7, and stores them in the external recording device (step S95c). The controller 11 then finishes the fourth data storing process and moves the process to step S10 of the log storing process.

In this case, the acquisition data are the associated data of the detection result by the human detecting sensor 191 and the detection result by the door open/close sensor 192; the detection result of the error in the behavior of the diffuser 18; the detection result of the behavior mode of the ozone sterilization device 100; measured ozone concentration; and the calculated CT value.

The external recording device may be the portable storage medium 200 inserted in the port 151, or it may be an external recording device connected by the wireless communication method via the communication unit 15.

If an abnormality has not been detected in the detection results (step S94c; NO), the controller 11 adds one to the number of the abnormality detection processes performed after the previous storage of the date and time of the storage timing and the acquisition data in the external recording device (step S96c).

Next, the controller 11 determines whether or not the abnormality detection process has been performed for a predetermined number of times or more (for example, 15 times or more) since the date and time of the storage timings and the acquisition data was previously stored in the external recording device (step S97c). The predetermined number of times can be set to any value.

If the abnormality detection process has been performed for a predetermined number of times or more (step S97c: YES), the controller 11 moves the process to step S95c.

In other words, the controller 11 (the acquisition unit) acquires the detection results by the human detecting sensor 191 and the detection results by the detector at the storage timings (at a predetermined time interval(s)). If the acquisition unit acquires the detection results that the human detecting sensor 191 has not detected human entry or exit and the detection result that the detector has not detected abnormalities continually for a predetermined number of times, the controller 11 (the storage controller) stores the acquisition data in the external recording device.

If the abnormality detection process has not been performed for a predetermined number of times yet (step S97c: NO), the controller 11 finishes the fourth data storing process and moves the process to step S10 of the log storing process.

Because the associated data (the acquisition data) are stored in the external recording device only when an abnormality has been detected in the detection results or when the abnormality detection process has been performed for a predetermined number of times or more without detection of abnormalities as described above, it is possible to reduce the frequency of communication between the ozone sterilization device 100 and the external recording device and to reduce the power consumption of the controller 11 (CPU).

Also, when there is an unusual change in the time series data of the ozone concentration, it is possible to identify the cause of the change in the ozone concentration based on the abnormality in the detection results, to reduce the amount of associated data (the acquisition data) stored in the external recording device, and to save capacity on the external recording device.

The ozone sterilization device 100 of the present embodiment described above includes the ozone generator 17 that generates ozone gas, the ozone concentration measurement unit 16 that measures the ozone concentration in the air, the communication unit 15 that performs data communication with the external recording device (the portable storage medium 200, wireless terminal 300, or data server 500), the acquisition unit (the controller 11) that acquires the acquisition data which is the ozone concentration measured by the ozone concentration measurement unit 16 at the predetermined acquisition timing, and the storage controller (the controller 11) that stores the acquisition data in the external recording device via the communication unit 15.

Therefore, the acquisition data acquired at the predetermined acquisition timings are stored in the external recording device, and the user can be provided with the data via the external recording device so as to manage sterilization with ozone more effectively.

The ozone sterilization device 100 according to the present embodiment includes the ozone generator 17 that generates ozone gas, the ozone concentration measurement unit 16 that measures the ozone concentration in the air, the time measurement unit (the controller 11) that measures the elapsed time from the start of ozone gas generation by the ozone generator 17, the calculator (the controller 11) that calculates the CT value based on the ozone concentration measured by the ozone concentration measurement unit 16 and the elapsed time measured by the time measurement unit, the communication unit 15 that performs data communication with the external recording device, the acquisition unit (the controller 11) that acquires the acquisition data which is the ozone concentration measured by the ozone concentration measurement unit 16 at the predetermined acquisition timing and/or the CT value calculated by the calculator, and the storage controller (the controller 11) that stores the acquisition data in the external recording device via the communication unit 15.

Therefore, the acquisition data acquired at the predetermined acquisition timings are stored in the external recording device, and the user can be provided with the data via the external recording device and can manage sterilization with ozone more effectively.

The ozone sterilization device 100 according to the present embodiment includes the human detecting sensor 191 that detects entry and exit of a human, the detector (the controller 11, the door open/close sensor 192) that detects abnormality in the ozone sterilization device 100, and the storage controller (the controller 11). The storage controller stores, in the external recording device, data in which at least one of the detection result by the human detecting sensor 191 and the detection result by the detector are associated with at least one of the ozone concentration measured by the ozone concentration measurement unit 16 and the CT value calculated by the calculator.

Therefore, when there is a change such as a temporary decrease in the ozone concentration in the time series data of the ozone concentration, it is possible to identify the cause of the decrease in the ozone concentration based on the detection result by the sensor 19, the detection result of an error in the behavior of the diffuser 18, and the detection result of the behavior mode of the ozone sterilization device 100.

In the ozone sterilization device 100 according to the present embodiment, the acquisition unit acquires the detection results by the human detecting sensor 191 and the detection results by the detector at predetermined time intervals. If the acquisition unit acquires the detection result that the human detecting sensor 191 has not detected human entry or exit and the detection result that the detector has detected no abnormality continually for a predetermined number of times, the storage controller stores the acquisition data in the external recording device.

As a result, it is possible to reduce the frequency of communication between the ozone sterilization device 100 and the external recording device and to reduce the power consumption of the controller 11 (CPU).

Also, when there is an unusual change in the time series data of the ozone concentration, it is possible to identify the cause of the change in the ozone concentration based on the abnormality in the detection results, to reduce the amount of associated data (the acquisition data) stored in the external recording device, and to save capacity on the external recording device.

The ozone sterilization device 100 according to the present embodiment includes the display 14 (the calculated CT value display 141) and the controller 11 that causes the display 14 to display the CT value calculated by the calculator.

Thus, the user can grasp the CT value calculated at a predetermined acquisition timing.

In the ozone sterilization device 100 according to the present embodiment, the storage controller stores the acquisition data in the external recording device continually at a predetermined time interval.

Thus, the user can be provided with the acquisition data in a format that allows calculation of the CT values and verification of the CT values.

In the ozone sterilization device 100 according to the present embodiment, the predetermined time interval is a time period in minute.

Since the CT value [ppm × min] is the product of the ozone concentration [ppm] and the ozone exposure time (fumigation time) [min], the ozone concentrations and CT values stored at minute intervals allow the user to use the stored ozone concentrations and CT values to further improve work efficiency in verifying the CT values.

In the ozone sterilization device 100 according to the present embodiment, the predetermined time interval can be set to any value.

Therefore, the user can be provided with the acquisition data acquired at a time interval that set to a desired value.

In the ozone sterilization device 100 according to the present embodiment, the communication unit 15 performs data communication by the wireless communication method.

When the acquisition data is stored in the external recording device by the wireless communication method, the user can easily acquire the stored acquisition data from the external recording device even when the ozone sterilization device 100 is placed at a high place that the user has difficulty in reaching.

In the ozone sterilization device 100 according to the present embodiment, the storage controller stores the acquisition data in a cloud server (the data server 500) as the external recording device.

Accordingly, a user can enter a password from a personal computer 600, log in to the data server 500, and view the data stored on the data server 500 from anywhere. Therefore, the user can check the stored acquisition data even when the user is not in the room where the ozone sterilization device 100 is placed.

In the ozone sterilization device 100 according to the present embodiment, the communication unit 15 performs data communication by the wired communication method.

Therefore, even when a device (for example, a medical device defined in Article 2 of the Act on Pharmaceuticals and Medical Devices Agency) that is poorly tolerant to near-field electromagnetic fields of an RF wireless communication device is placed in the room where the ozone sterilization device 100 is placed, the acquisition data can be stored without affecting the device.

In the ozone sterilization device 100 according to the present embodiment, the storage controller stores the acquisition data in the portable storage medium 200 as the external recording device.

Therefore, the user can remove only the portable storage medium 200 where the acquisition data are stored from the ozone sterilization device 100 and verify the CT value in a place outside of the room where the ozone sterilization device 100 is placed.

The ozone sterilization device 100 according to the present embodiment includes the lid 31 that covers the portable storage medium 200 and the main housing 30 to which the lid 31 is attached. The lid 31 can be detached from the main housing 30 using the dedicated tool.

Therefore, the portable storage medium 200 containing data cannot be easily removed even when the ozone sterilization device 100 is placed in an environment where an unspecified large number of people are present.

In the ozone sterilization device 100 according to the present embodiment, the ozone generator 17 generates ozone gas at a concentration applicable to a manned environment.

When the ozone sterilization device 100 runs continuously for a long time to generate a low concentration of ozone gas that is applicable to a manned environment, the user can correctly grasp, for example, a situation where the ozone concentration has temporarily decreased.

In the ozone sterilization device 100 according to the present embodiment, the storage controller outputs the communication clock signal only when the communication unit 15 performs data communication with the external recording device.

Therefore, the power consumption of the controller 11 (CPU) can be suppressed.

In the ozone sterilization device 100 according to the present embodiment, the storage controller collectively stores the acquisition data acquired multiple times during the predetermined period in the external recording device.

Therefore, it is possible to reduce the frequency of communication between the ozone sterilization device 100 and the external recording device and to reduce the power consumption of the controller 11 (CPU).

The above-described embodiments are preferred examples of the ozone sterilization device 100 according to the present invention and do not intended to limit the present invention.

For example, the sensor 19 in the above embodiment includes the human detecting sensor 191 and the door open/close sensor 192, but may also include other sensors.

In step S92a of the second data storing process in Modification Example 2 and in step S92c of the fourth data storing process in Modification Example 4 of the above embodiments, the controller 11 acquires the detection result of whether or not an error has occurred in the behavior of the diffuser 18, but the present invention is not limited to this. The controller 11 may acquire the detection result of whether or not an error has occurred in a part other than the diffuser 18 of the ozone sterilization device 100.

Although embodiments of the present invention have been described and illustrated in detail, the disclosed embodiments are made for purposes of illustration and example only and not limitation. The scope of the present invention should be interpreted by terms of the appended claims.

## Claims

1. An ozone sterilization device comprising:
an ozone generator that generates ozone gas;
an ozone concentration measurement unit that measures an ozone concentration in air;
a communication unit that performs data communication with an external recording device;
an acquisition unit that acquires acquisition data at a predetermined acquisition timing, the acquisition data including the ozone concentration measured by the ozone concentration measurement unit; and
a storage controller that stores the acquisition data in the external recording device via the communication unit.

2. An ozone sterilization device comprising:
an ozone generator that generates ozone gas;
an ozone concentration measurement unit that measures an ozone concentration in air;
a time measurement unit that measures an elapsed time from start of generating ozone gas by the ozone generator;
a calculator that calculates a CT value based on the ozone concentration measured by the ozone concentration measurement unit and the elapsed time measured by the time measurement unit;
a communication unit that performs data communication with an external recording device;
an acquisition unit that acquires acquisition data at a predetermined acquisition timing, the acquisition data including at least one of the ozone concentration measured by the ozone concentration measurement unit and the CT value calculated by the calculator; and
a storage controller that stores the acquisition data in the external recording device via the communication unit.

3. The ozone sterilization device according to claim 2, further comprising:
a human detecting sensor that detects entry and exit of a human; and
a detector that detects an abnormality in the ozone sterilization device,
wherein the storage controller stores, as the acquisition data, data in which the at least one of a detection result by the human detecting sensor and a detection result by the detector are associated with the at least one of the ozone concentration measured by the ozone concentration measurement unit and the CT value calculated by the calculator in the external recording device.

4. The ozone sterilization device according to claim 3,
wherein the acquisition unit acquires a detection result by the human detecting sensor and a detection result by the detector at a predetermined time interval, and
wherein, upon the acquisition unit acquiring a detection result that the human detecting sensor has not detected human entry or exit and a detection result that the detector has not detected an abnormality continually for a predetermined number of times, the storage controller stores the acquisition data in the external recording device.

5. The ozone sterilization device according to any one of claims 2 to 4, further comprising:
a display; and
a controller that causes the display to display the CT value calculated by the calculator.

6. The ozone sterilization device according to any one of claims 1 to 5,
wherein the storage controller stores the acquisition data in the external recording device continually at a predetermined time interval.

7. The ozone sterilization device according to claim 6,
wherein the predetermined time interval is a time period in minute.

8. The ozone sterilization device according to claim 6 or 7,
wherein the predetermined time interval is set to any value.

9. The ozone sterilization device according to any one of claims 1 to 8,
wherein the communication unit performs data communication by a wireless communication method.

10. The ozone sterilization device according to claim 9,
wherein the storage controller stores the acquisition data in a cloud server as the external recording device.

11. The ozone sterilization device according to any one of claims 1 to 8,
wherein the communication unit performs data communication by a wired communication method.

12. The ozone sterilization device according to claim 11,
wherein the storage controller stores the acquisition data in a portable storage medium as the external recording device.

13. The ozone sterilization device according to claim 12, further comprising:
a lid that is configured to cover the portable storage medium; and
a main housing to which the lid is attached,
wherein the lid is detachable from the main housing using a dedicated tool.

14. The ozone sterilization device according to any one of claims 1 to 13,
wherein the ozone generator generates ozone gas at a concentration applicable to a manned environment.

15. The ozone sterilization device according to any one of claims 1 to 14,
wherein the storage controller outputs a communication clock signal only when the communication unit performs data communication with the external recording device.

16. The ozone sterilization device according to any one of claims 1 to 15,
wherein the storage controller collectively stores the acquisition data acquired multiple times during a predetermined period in the external recording device.
